# EUROPEAN PATENT SPECIFICATION

(11) **EP 0 420 556 B1**
(45) Date of publication and mention of the grant of the patent: **17.08.1994**
(21) Application number: 90310438.8
(22) Date of filing: 24.09.1990
(51) Int. Cl.: C07C 43/225, C07C 41/22, B41M 5/136, B41M 5/30

(54) **Process for the preparation of 4-bromo-3-alkylphenyl ethers**
Verfahren zur Herstellung von 4-Brom-3-Alkylphenylethern
Procédé pour la préparation d'éthers 4-bromo-3-alkylphényliques

(30) Priority: 27.09.1989 US 413886
(43) Date of publication of application: 03.04.1991
(73) Proprietor: ESCO Company Limited Partnership, Muskegon, Michigan 49443 (US)
(72) Inventor: Filius, Larry L., Muskegon, Michigan 49441 (US)
(74) Representative: Farwell, William Robert

(56) References cited:
- JOURNAL OF THE CHEMICAL SOCIETY, PERKIN TRANSACTIONS I, 1983, pages 625-631; A.J. PEARSON et al.: "Organoiron complexes in organic synthesis. Part 27. Synthesis and reactivity of tricarbonyliron derivatives of 1,2-disubstituted 4-alkoxycyclohexadienylium cations"
- BULLETIN OF THE CHEMICAL SOCIETY OF JAPAN, vol. 62, no. 2, February 1989, pages 591-593; H. KONISHI et al.: "A mild selective monobromination reagent system for alkoxybenzenes; N-bromosuccinimide-silica gel"
- JOURNAL OF THE CHEMICAL SOCIETY, SECTION D, CHEMICAL COMMUNICATIONS, no. 24, 22nd December 1971, pages 1596-1597; B.J. AURET et al.: "The NIH shift during the hydroxylation of aromatic substrates by fungi"
- CHEMICAL ABSTRACTS, vol. 81, no. 21, 25th November 1974, page 382, abstract no. 135603s, Columbus, Ohio, US; E.A. MAWDSLEY et al.: "Synthesis of some substituted bromoanisoles"
- CHEMICAL ABSTRACTS, vol. 104, no. 13, 31st March 1986, page 682, abstract no.109187h, Columbus, Ohio, US; H. SENSUI et al, "Diphenylamines"; & JP-A-60 214 763

## Description

The invention relates to chemical intermediates, especially for colour formers for copy papers.

### General

Bromination of aromatic compounds, and more particularly bromination of anisole compounds particularly alkylated anisoles, is established practice. These brominated compounds are commercially useful in the manufacture of a variety of products, particularly in the synthesis of certain organic compounds such as various dyes used as colour formers for copy papers and for other purposes.

Present methods for bromination of 3-alkylanisoles, particularly 3-methylanisole, involve liquid reaction of bromine and the alkyl anisole. Reaction is typically conducted at elevated temperature with one or both reactants dissolved in a solvent such as heptane, carbon tetrachloride, acetic acid or aqueous hydrogen bromide solution of approximately 48% HBr.

German Offenlegungsschrift 29 19 234 (application date November 20, 1980) discloses a process for manufacture of p-bromoanisole by reacting anisole with a solution of bromine in an aqueous hydrogen bromide solution. The '234 process is said to reduce 2,4-dibromoanisole proportions. Earlier proce '234 application including solvent free reaction relying on an iron catalyst, and including reaction of anisole in glacial acetic acid as solvent. These prior art products are characterized as contaminated with considerable amounts of 2, 4-dibromoanisole.

The '234 application while improving over the earlier methods still has the drawback of dibromoanisole impurities in amounts more than are desirable (2.5% by weight).

Prior art methods generally have dibromoanisole contamination levels of 2.5 to 10%, though the reference Bulletin Chemical Society Japan 591-593 62 (1989) refers to N-bromosuccinimide in the presence of silica gel giving para bromination of methoxybenzene at above 98.5% yield.

It would be an advance in the art if a method were found to appreciably reduce dibromo contamination in the manufacture of 4-bromo-3-alkyl phenol ethers from 3-alkyl phenol ethers, using bromine.

### Invention

The invention provides a method for making a 4-bromo-3-alkyl phenol ether in particular 4-bromo-3-alkylanisole comprising introducing the corresponding 3-alkyl phenol ether into a reactor having a reaction zone, establishing the 3-alkyl phenol ether as a vapor in the reaction zone, introducing bromine vapor to the reaction zone and into the presence of the vaporized 3-alkyl phenol ether to form mixed vapors, and maintaining the mixed vapors in the vapor phase for a time and at a temperature sufficient to convert the 3-alkyl phenol ether to 4-bromo-3-alkyl phenol ether.

Advantageous features of the method are set out below and in the claims hereafter to which reference may be made, but briefly the reaction zone pressure is preferably 1.33 to 26.6 Kilopascals (10 mm to 200 mm mercury), advantageously around 6.65 Kilopascals (50 mm Hg), the temperature is less than 100^{o}C, and the mixed vapours are maintained by reflux of the 3-alkyl phenol ether.

The disclosed method has the advantage of that it eliminates solvent diluent, increases productivity, and eliminates or minimizes need for subsequent produce purification.

### Detailed Description

The invention is particularly applicable to the making of 4-bromo-3-methylanisole 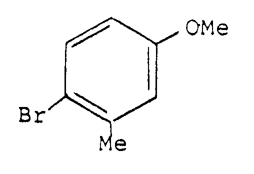
but the 3-alkyl group can be selected to be other lower alkyl groups particularly of 8 carbons or less, preferably 5 carbons or less. The methoxy group of anisole may similarly be replaced by other lower alkoxy groups.

In preferred form the method comprises establishing a molar excess of 3-alkylanisole as a vapor in a reaction zone of a reactor, for example at 1.33 to 26,6 Kilopascals (10 mm to 200 mm mercury pressure), desirably around 6,65 Kilopascals (50 mm). Bromine (58^{o}C) is established as a vapor in the reactor and introduced to the reaction zone commingling with the established 3-alkylanisole vapor. The 3-alkylanisole vapor enters the reaction zone which being at a slightly higher temperature of however, desirably, less than 100 ^{o}C, insures complete vaporization and the resultant production of 4-bromo-3-alkylanisole with almost no dibrominated impurity.

Vaporization of the 3-alkylanisole can be accomplished by conventional means such as application of heat, and/or reduction of reactor pressure, preferably both steps. Bromine vapor is generated from a bromine source such as by heating liquid Br₂ above its boiling point 58^{o}C to form a gas. The bromine vapor is directed to and introduced to the reaction zone of the reactor wherein the vaporized 3-alkylanisole is maintained in a gaseous phase. Such maintenance of 3-alkyl anisole in the gaseous phase is readily accomplishable by arranging reflux of the partially reacted reaction zone effluent to a rectifying column, continued heat input to the reboiler, and maintenance of the reaction zone at about 10^{o} to 30^{o}C higher temperature than the entering vapor. The resulting mixed vapors are maintained in the vapor phase, such as via reflux, for a time sufficient to react and convert the 3-alkylanisole to 4-bromo-3-alkylanisole.

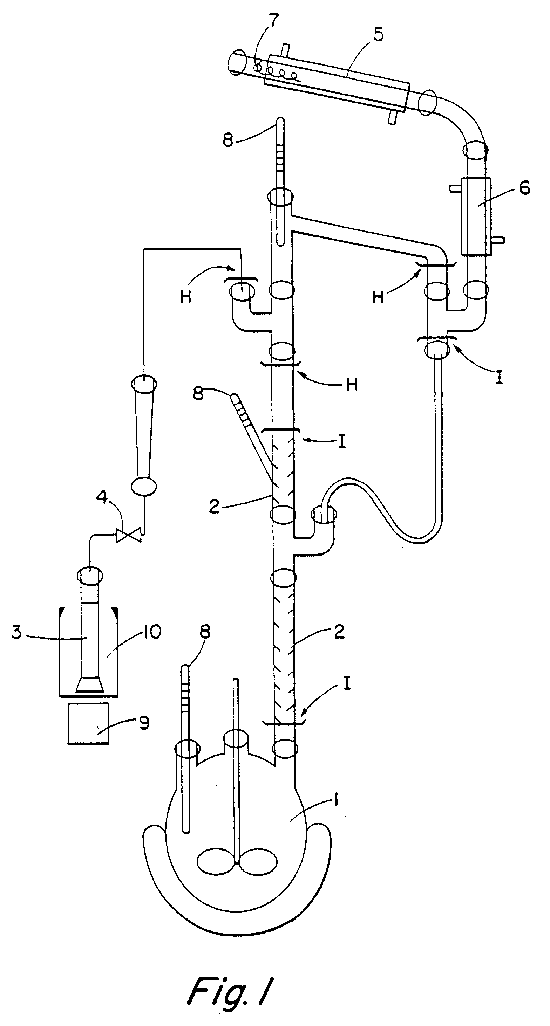

Looking now at Fig. 1, a convenient reactor apparatus is depicted for effecting vapor-phase anisole bromination according to the invention.

Round bottom flask 1 is provided with a thermometer 8 and stirrer and a distillation or reflux column 2. A bromine source 3 stirred by magnetic mixer 9 is maintained in a water bath 10 and via control valve 4 gas is led ultimately to the top of reflux column 2.

A vacuum is applied via condenser 5 which uses a cooling fluid of glycol at -15^{o}C. The condenser can be fitted with a glass wool demister. An optimal water condenser 6 can also be included. The 3-alkylanisole is introduced to flask 1 and heated from about 90^{o} to 150^{o}C. The bromine source 3 is heated via a water bath. Column 2 is preferably maintained at about 90^{o} - 100^{o}C when the 3-alkylanisole is selected to be 3-methylanisole. The above temperatures are based on about 6,65 Kilopascals (50mm) Hg pressure for the system. Higher applied vacuum of course would alter these recited temperature ranges.

Points between the areas marked H have a heat tape applied. Points between areas marked I are insulated.

### Example

Using an apparatus such a depicted in Fig. 1, a round bottom flask 1 reboiler is charged with 329g of neat 3-methylanisole. This charge can be varied within the capacity limits of the reboiler.

A bromine vaporizor is initially charged with 100 mls of bromine (its capacity) and later with the remaining 28.5 mls.

The system is reduced to a pressure of 50mm Hg, and the reboiler is heated to effect reflux at about 5.5 mls/minute. The preferred reflux rate range is about 1 ml to about 8 mls/minute. The vapor reactor heat tape voltage is adjusted just high enough to prevent condensation of 3-methylanisole in the vapor reactor. Then the Br₂ feed valve is opened and adjusted to give 0.2 mls of vapor per minute. The preferred range is up to 0.5 mls/minute at the recommended system pressure of 50mm Hg.

As the bromination progresses the temperature of the upper part of the column remains constant (at about 90^{o}C) while the reboiler temperature slowly rises from about 97^{o}C.

When the reaction is just about done, the column temperature will begin to rise. At this time the Br₂ addition rate is decreased to maintain a column temperature of less than 100^{o}C (10^{o} max. temperature rise for the column). When the column temperature exceeds a 10^{o}C differential and the Br₂ feed rate is at the lowest practical rate, the reaction is terminated; the reboiler temperature at this time is about 150^{o}C.

The total reaction time with this equipment and charge is 11 hours. The crude product weight is 522g, and the residual 3-methylanisole (about 1% to 5%) is distilled off with high reflux and recycled to the next bromination reaction. A typical precut is 4.6% of crude weight or 24g which contains about 22g of 3-methylanisole to be recycled. The remaining product (498g) assays:
98% 4-bromo-3-methyl-anisole
0.1% dibromo anisole
1.0% 3-methyl-anisole dimers and trimers
0.9% other impurities including bromo cresol (from cresol impurity in the 3-methyl-anisole)

The final assayed yield is 488g from 307g of 3-methylanisole reacted or 96.6% reaction yield.

The principles, preferred embodiments, and modes of operation of the present invention have been described in the foregoing specification. The invention which is intended to be protected herein, however, is not to be construed as limited to the particular forms disclosed, since these are to be regarded as illustrative rather than restrictive. Variations and changes can be made by those skilled in the art without departing from the spirit and scope of the invention, within the claims.

## Claims

1. A method for making a 4-bromo-3-alkyl phenol ether in particular 4-bromo-3-alkylanisole comprising introducing the corresponding 3-alkyl phenol ether into a reactor having a reaction zone, establishing the 3-alkyl phenol ether as a vapor in the reaction zone, introducing bromine vapor to the reaction zone and into the presence of the vaporized 3-alkyl phenol ether to form mixed vapors, and maintaining the mixed vapors in the vapor phase for a time and at a temperature sufficient to convert the 3-alkyl phenol ether to the 4-bromo-3-alkyl phenol ether.

2. The method according to claim 1 wherein the reaction zone of the reactor is maintained at a pressure of from 1.33 to 26.6 Kilopascals (10mm to 200mm mercury).

3. The method according to claim 2 wherein the reaction zone of the reactor is maintained at about 6.65 Kilopascals (50mm Hg).

4. The method according to any preceding claim wherein maintaining the mixed vapors in a vaporized phase is accomplished by refluxing the 3-alkyl phenol ether in the reactor.

5. The method according to any preceding claim wherein the temperature of the reaction zone is maintained at less than 100^{o}C.

6. The method according to any preceding claim wherein the 4-bromo-3-alkyl phenol ether made is the 3-methyl compound.

## Patentansprüche

1. Verfahren zur Herstellung eines 4-Brom-3-Alkylphenolethers, insbesondere von 4-Brom-3-Alkylanisol, das die folgenden Schritte umfaßt: Einleiten des entsprechenden 3-Alkylphenolethers in einen Reaktor mit einer Reaktionszone, Umwandeln des 3-Alkylphenolethers in einen Dampf in der Reaktionszone, Einleiten von Bromdampf in die Reaktionszone und in die Umgebung des eingedampften 3-Alkylphenolethers zur Bildung von Mischdamämpfen, und Halten der Mischdämpfe in der Dampfphase für eine Zeitspanne und bei einer Temperatur, die zur Umsetzung des 3-Alkylphenolethers in 4-Brom-3-Alkylphenolether ausreicht.

2. Verfahren nach Anspruch 1, wobei die Reaktionszone des Reaktors bei einem Druck von 1,33 bis 26,6 Kilopascal (10 mm bis 200 mm Quecksilbersäule) gehalten wird.

3. Verfahren nach Anspruch 2, wobei die Reaktionszone des Reaktors bei etwa 6,65 Kilopascal (50 mm Hg) gehalten wird.

4. Verfahren nach einem der vorhergehenden Ansprüche, wobei das Halten der Mischdämpfe in einer eingedampften Phase durch Rückfließenlassen des 3-Alkylphenolethers in den Reaktor erfolgt.

5. Verfahren nach einem der vorhergehenden Ansprüche, wobei die Temperatur der Reaktionszone auf weniger als 100°C gehalten wird.

6. Verfahren nach einem der vorhergehenden Ansprüche, wobei das hergestellte 4-Brom-3-Alkylphenolether die 3-Methylverbindung ist.

## Revendications

1. Procédé pour la préparation d'un éther 4-bromo-3-alkylphénylique en particulier d'un 4-bromo-3-alkylanisole comprenant les étapes consistant à introduire un éther 3-alkylphénilyque correspondant dans un réacteur ayant une zone de réaction, porter l'éther 3-alkylphénilyque sous forme de vapeur dans la zone de réaction, introduire de la vapeur de brome dans la zone de réaction et en présence de l'éther 3-alkylphénilyque pour former un mélange de vapeurs, et maintenir le mélange de vapeurs en phase vapeur pendant un temps et à une température suffisantes pour transformer l'éther 3-alkylphénilyque en un éther 4-bromo-3-alkylphénylique.

2. Procédé selon la revendication 1 dans lequel la zone de réaction du réacteur est maintenue à une pression comprise entre 1,33 et 26,6 Kilopascals (10mm à 200mm de mercure).

3. Procédé selon la revendication 2 dans lequel la zone de réaction duréacteur est maintenue à environ 6,65 Kilopascals (50mm de mercure).

4. Procédé selon l'une quelconque des revendications précédentes dans lequel le maintien du mélange de vapeurs en phase vapeur est réaliser en chauffent à reflux l'éther 3-alkylphénilyque dans le réacteur.

5. Procédé selon l'une quelconque des revendications précédentes dans lequel la température de la zone de réaction est maintenue inférieure à 100°C.

6. Procédé selon l'une quelconque des revendications précédentes dans lequel l'éther 4-bromo-3-alkylphénylique préparé est le composé 3-méthyle.
